(19) Europäisches Patentamt
European Patent Office
Office européen des brevets

(11) EP 1 862 446 A2

(12) **EUROPÄISCHE PATENTANMELDUNG**

(43) Veröffentlichungstag:
**05.12.2007 Patentblatt 2007/49**

(51) Int Cl.:
***C07C 209/40*** *(2006.01)* ***C07C 239/20*** *(2006.01)*
***C07C 229/34*** *(2006.01)*

(21) Anmeldenummer: **07107989.1**

(22) Anmeldetag: **11.05.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK YU**

(30) Priorität: **02.06.2006 DE 102006026329**

(71) Anmelder: **Evonik Degussa GmbH**
**40474 Düsseldorf (DE)**

(72) Erfinder:
- **Kadyrov, Renat**
  **60389, Frankfurt (DE)**
- **Riermeier, Thomas**
  **64372, Ober-Ramstadt (DE)**
- **Almena, Juan**
  **63454, Hanau (DE)**
- **Monsees, Axel**
  **60487, Frankfurt (DE)**
- **Groß, Peter**
  **65451, Kelsterbach (DE)**
- **Rossen, Kai**
  **63452, Hanau (DE)**

(54) **Verfahren zur Herstellung von enantiomerenangereicherten Hydroxylaminderivaten**

(57) Die vorliegende Erfindung ist auf ein Verfahren zur Herstellung von N-Hydroxylaminderivaten gerichtet. Diese Verbindungen lassen sich leicht zum Beispiel durch hydrogenolytische Spaltung in die entsprechenden Amine umwandeln. Derartige Amine sind wichtige Intermediate in der chemischen (Groß)Synthese.

Das vorliegende Verfahren bezieht sich insbesondere auf ein Verfahren zur Herstellung von enantiomerenangereicherten N-Hydroxylaminderivaten ausgehend von Oximderivaten der allgemeinen Formel (I).

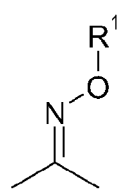

(I)

EP 1 862 446 A2

## Beschreibung

Die vorliegende Erfindung betrifft ein Verfahren zur Herstellung von Hydroxylaminderivaten. Insbesondere richtet sich die vorliegende Erfindung u.a. auf ein Verfahren zur Herstellung von enantiomerenangereicherten Hydroxylaminderivaten durch Hydrierung von speziellen O-substituierten Oximen. Diese N-Oxyamine können im Anschluss stereokonservativ zu den entsprechenden Aminen weiter reduziert werden.

N-Hydroxylamine und insbesondere enantiomerenangereicherte N-Hydroxylamine sind wertvolle Intermediate in der organischen Synthese im Labormaßstab als auch für die industrielle Produktion von z.B. Wirkstoffen (siehe z.B. H. C. J. Ottenheim, J. D. M. Herscheid Chem. Rev. 1986, 86, 697-707) oder Initiatoren von Radikalpolymerisation. Die Herstellmethoden, die dem Fachmann in diesem Bereich bekannt sind, sind in der Übersicht (B. Zeeh , H. Metzger in Methoden der Organische Chemie ( Houben - Weyl ) Bd. X / 1 , 1971 , Georg Thieme Verlag , Stuttgart , S. 1092 - 1279)) zusammengefasst. Optisch aktive N-Hydroxylamine wurden in letztere Zeit auch durch asymmetrische Hydrosilylierung (S.-I. Murahashi, S. Watanabe, T. Shiota Chem Commun.,1994, 725-726) und Hydrierung (S.-I. Murahashi, T. Tsuji, S. Ito Chem Commun., 2000, 409-410) ausgehend von Nitronen hergestellt.

Bei der Reduktion von Oximen entstehen in der Regel Amine (H. Metzger in Methoden der Organische Chemie (Houben - Weyl) Bd. X / 4 , 1968 , Georg Thieme Verlag , Stuttgart , S. 236-237; T. Munegumi, K. Harada Bull. Chem. Soc. Jpn., 1988, 61, 1425-1427; E. Breitner, E. Roginski, P.N. Rylander J. Chem. Soc. 1959, 2918-2920; W. Hartung, J. Am. Chem. Soc., 1928, 50, 3370-3374). Auch homogen katalysierte Hydrierungen von Oximen liefern Aminen als Produkte. (A.S.C. Chan, C.-C. Chen, C.-W. Lin, Y.-C. Lin, M.-C. Cheng, S.-M. Peng J. Chem. Soc. Chem. Commun. 1995, 1767-8; P. Krasik, H. Alper, Tetrahedron: Asymmetry, 1992, 3, 1283-8; K. Yamamoto, Saeed-Ur-Rehman Chem. Lett. 1984, 1603-6; Y. Xie, A. Mi, Y. Jiang, H. Liu Synth. Commun., 2001, 31, 2767-2771).

Es besteht daher ein Bedarf an Verfahren zur reduktiven Herstellung von N-Hydroxylaminen ausgehend von Oximen.

Aufgabe der vorliegenden Erfindung war es deshalb, ein Verfahren anzugeben, mit dem Oxime in guten Ausbeuten in die entsprechenden Hydroxylaminderivate umgesetzt werden können. Insbesondere sollte das erfindungsgemäße Verfahren vom ökonomischen wie ökologischen Standpunkt aus betrachtet den Verfahren des Standes der Technik überlegen sein und es insbesondere erlauben, die Hydroxylaminderivate ggf. auch enantioselektiv herstellen zu können.

Diese und weitere nicht näher spezifizierte, sich jedoch aus dem Stand der Technik in nahe liegender Weise ergebende Aufgabenstellungen werden durch ein Verfahren mit den Merkmalen des gegenständlichen Anspruchs 1 gelöst. Ansprüche 2 bis 10 bilden bevorzugte Ausgestaltungen des erfindungsgemäßen Verfahrens.

Dadurch, dass man in einem Verfahren zur Herstellung von Hydroxylaminderivaten diese durch Hydrierung von Oximderivaten aufweisend ein oder mehrere Strukturelemente der allgemeinen Formel (I)

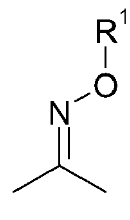

(I)

worin

$R^1$ bzw. $R^{1'}$ bedeutet

$(C_1-C_8)$ -Alkyl, HO- $(C_1-C_8)$ -Alkyl, $(C_2-C_8)$ -Alkoxyalkyl, $(C_6-C_{18})$ -Aryl, $(C_7-C_{19})$ -Aralkyl, $(C_3-C_{18})$ -Heteroaryl, $(C_4-C_{19})$ - Heteroaralkyl, $(C_1-C_8)$ -Alkyl- $(C_6-C_{18})$ -Aryl, $(C_1-C_8)$ -Alkyl-$(C_3-C_{18})$ -Heteroaryl, $(C_3-C_8)$ -Cycloalkyl, $(C_1-C_8)$ -Alkyl-$(C_3-C_8)$ -Cycloalkyl, $(C_3-C_8)$ -Cycloalkyl- $(C_1-C_8)$ -Alkyl, oder $R^1$ ist $C(O)R^{1'}$ oder $C(O)OR^{1'}$ oder $C(O)NHR^{1'}$ oder $C(O)NR^{1'}_2$ oder $P(O)R^{1'}_2$ oder $P(O)(OR^{1'})_2$ oder $SO_{(3-1)}$-$R^{1'}$ oder $SiR^{1'}R^{1'}R^{1'}$ oder ist $(C_1-C_8)$-Alkyl-$C(O)R^{1'}$ oder $(C_1-C_8)$-Alkyl-$C(O)OR^{1'}$ oder $(C_1-C_8)$-Alkyl-$C(O)NHR^{1'}$ oder $(C_1-C_8)$-Alkyl-$C(O)NR^{1'}_2$ oder $(C_1-C_8)$-Alkyl-$P(O)R^{1'}_2$ oder $(C_1-C_8)$-Alkyl-$P(O) (OR^{1'})_2$ oder $(C_1-C_8)$-Alkyl-$SO_{(3-1)}$-$R^{1'}$ oder $(C_1-C_8)$-Alkyl-$SiR^{1'}R^{1'}R^{1'}$,

in Gegenwart von homogen löslichen Übergangsmetallkatalysatoren herstellt, gelangt man völlig überraschend, dafür aber nicht minder vorteilhaft zur Lösung der gestellten Aufgabe. Die angesprochenen Oximderivate lassen sich entgegen den Vermutungen des Standes der Technik durch Übergangsmetallkatalysatoren in beachtlichen Ausbeuten und Einsatz

von chiralen Liganden mit zum Teil hohen Enantiomerenüberschüssen zu N-Hydroxylaminderivaten hydrieren.

Bevorzugt ist ein Verfahren, bei dem man Oxime der allgemeinen Formel (II)

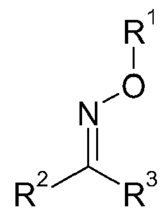

(II)

worin

$R^1$ bevorzugt ($C_1$-$C_8$) -Alkyl bedeutet,

$R^2$ und $R^3$ unabhängig voneinander bedeuten

H, ($C_1$-$C_8$) -Alkyl, HO- ($C_1$-$C_8$) -Alkyl, ($C_2$-$C_8$) -Alkoxyalkyl, ($C_6$-$C_{18}$)- Aryl, ($C_7$-$C_{19}$) -Aralkyl, ($C_3$-$C_{18}$) -Heteroaryl, ($C_4$-$C_{19}$) - Heteroaralkyl, ($C_1$-$C_8$) -Alkyl- ($C_6$-$C_{18}$) -Aryl, ($C_1$-$C_8$) -Alkyl-($C_3$-$C_{18}$)-Heteroaryl, ($C_3$-$C_8$) -Cycloalkyl, ($C_1$-$C_8$)-Alkyl-($C_3$-$C_8$) -Cycloalkyl, ($C_3$-$C_8$)-Cycloalkyl-($C_1$-$C_8$)-Alkyl, oder $R^2$ bzw. $R^3$ bedeuten C(O)$R^{1'}$ oder C(O)O$R^{1'}$ oder C(O) NH$R^{1'}$ oder C (O) N$R^{1'}_2$ oder P(O)$R^{1'}_2$ oder P(O) (O$R^{1'}$)$_2$ oder $SO_{(3-1)}$-$R^{1'}$ oder Si$R^{1'}R^{1'}R^{1'}$ oder bedeuten ($C_1$-$C_8$) -Alkyl-C (O) $R^{1'}$ oder ($C_1$-$C_8$) - Alkyl-C(O)O$R^{1'}$ oder ($C_1$-$C_8$)-Alkyl-C(O)NH$R^{1'}$ oder ($C_1$-$C_8$) - Alkyl-C(O)N$R^{1'}_2$ oder ($C_1$-$C_8$)-Alkyl-P(O)$R^{1'}_2$ oder ($C_1$-$C_8$) - Alkyl-P (O) (O$R^{1'}$)$_2$ oder ($C_1$-$C_8$)-Alkyl-$SO_{(3-1)}$-$R^{1'}$ oder ($C_1$-$C_8$) - Alkyl-Si$R^{1'}R^{1'}R^{1'}$, oder

$R^2$ und $R^3$ und/oder $R^1$ und $R^2$ und/oder $R^1$ und $R^3$ bedeuten eine Verknüpfung durch eine kovalente Bindung, insbesondere eine ($C_2$-$C_8$) -Alkylenbrücke,

in die Reaktion einsetzt.

Ganz besonders bevorzugt ist der Einsatz von Oximen, bei denen $R^1$ Methyl oder Ethyl sind. Äußerst bevorzugt sind solche Oxime, die bei der erfindungsgemäßen Reaktion in $\alpha$-oder $\beta$-Aminosäuren umgesetzt werden können. Weiterhin besonders bevorzugt sind Oxime, bei denen $R^2$ bzw. $R^3$ ($C_1$-$C_8$) -Alkyl, HO- ($C_1$-$C_8$) -Alkyl, ($C_2$-$C_8$) -Alkoxyalkyl, ($C_6$-$C_{18}$) - Aryl, ($C_7$-$C_{19}$) -Aralkyl, ($C_3$-$C_{18}$)-Heteroaryl, ($C_4$-$C_{19}$) - Heteroaralkyl, ($C_1$-$C_8$) -Alkyl- ($C_6$-$C_{18}$) -Aryl, ($C_1$-$C_8$)-Alkyl-($C_3$-$C_{18}$)-Heteroaryl, ($C_3$-$C_8$) -Cycloalkyl, ($C_1$-$C_8$) -Alkyl- ($C_3$-$C_8$) -Cycloalkyl, ($C_3$-$C_8$)-Cycloalkyl-($C_1$-$C_8$)-Alkyl bedeutet.

Wie eben schon angesprochenen lassen sich die bei der erfindungsgemäßen Reaktion eingesetzten Oximderivate auch enantioselektiv reduzieren. Bewerkstelligt wird diese enantioselektive Hydrierung, indem man das zu reduzierende Oximderivate in Gegenwart der mit chiralen Liganden modifizierten Übergangsmetallkatalysatoren mit Wasserstoff oder einer den Wasserstoff liefernden Vorstufe umsetzt. Derartige chirale Liganden sind dem Fachmann bekannt (Ojima, I. Catalytic Asymmetric Synthesis 2000, 2. Auflage, Wiley-VCH Verlag GmbH). Bevorzugte Liganden sind solche ausgewählt aus der Gruppe der Phosphor- und Stickstoffhaltigen Liganden.

Besonders bevorzugte Liganden sind ausgewählt aus der Gruppe bestehend aus Diphosphanliganden.

Ganz besonders bevorzugt zum Einsatz kommen chirale Liganden ausgewählt aus der Gruppe der Ferrocenyldiphosphine, insbesondere mit der folgenden Struktur:

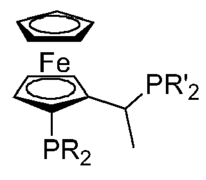

Sofern man die erfindungsgemäße Reaktion symmetrisch durchführen möchte, kann der Fachmann Liganden für die Übergangsmetallkatalysatoren wählen, die aus der Gruppe bestehend aus Phosphanen (Brunner, I. Hydrogenation in Applied Homogeneous Catalysis with organometallic Compounds, Eds. B. Cornils, W.A. Herrmann , Second Edition, Wiley-VCH Verlag GmbH, Weinheim, 2002, pp. 195-212).) entnommen werden.

Der Fachmann ist frei in seiner Wahl des Zentralatoms des zum Einsatz kommenden Übergangsmetallkatalysators. Der Fachmann nimmt für die gegenständliche Reaktion daher bevorzugt Übergangsmetalle ausgewählt aus

der Gruppe bestehend aus Ru, Os, Co, Rh, Ir, Ni, Pt, Pd, Fe, Cu als zentrale Atome der Übergangsmetallkatalysatoren. Besonders bevorzugt sind die Metalle Ru, Rh, Ir in diesem Zusammenhang.

Die erfindungsgemäße Hydrierung der oben angesprochenen Oximderivate kann mittels Hydrierung mit Wasserstoffgas oder mittels Transferhydrierung durchgeführt werden. Beide Methoden sind dem Fachmann hinlänglich vertraut (Transferhydrierung: "Asymmetric transferhydrogenation of C=O and C=N bonds", M. Wills et al. Tetrahedron: Asymmetry 1999, 10, 2045; "Asymmetric transferhydrogenation catalyzed by chiral ruthenium complexes" R. Noyori et al. Acc. Chem. Res. 1997, 30, 97; "Asymmetric catalysis in organic synthesis", R. Noyori, John Wiley & Sons, New York, 1994, S.123; "Transition metals for organic Synthesis" Ed. M. Beller, C. Bolm, Wiley-VCH, Weinheim, 1998, Bd.2, S. 97; "Comprehensive Asymmetrie Catalysis" Ed.: Jacobsen, E.N.; Pfaltz, A.; Yamamoto, H., Springer-Verlag, 1999; Hydrierung: "Comprehensive Asymmetric Catalysis" Ed.: Jacobsen, E.N.; Pfaltz, A.; Yamamoto, H., Springer-Verlag, 1999; "Applied Homogeneous Catalysis with Organometallic Compounds" Ed. B. Cornils, W. A. Herrmann, Willey-VCH, 2002).

Sofern der Fachmann die gegenständliche Reaktion in Gegenwart von Wasserstoffgas durchführt, stellt er vorzugsweise einen Wasserstoffdruck von 0,1 bis 100 bar, vorzugsweise 0,5 bis 50 bar, ein. Bevorzugt wird bei einem Wasserstoffdruck von 1,0 bis 30 bar hydriert.

Die Temperaturen, die während der Reaktion eingestellt wird, sind vom Fachmann nach seinem fachmännischen Können zu wählen. Der Fachmann orientiert sich bei der Einstellung der Temperaturen an der Tatsache, dass ein Ausgleich zwischen möglichst hohem Umsatz und gegebenenfalls hohen Enantiomerenüberschüssen und der Dauer der Reaktion sowie eine möglichst geringe Nebenproduktspektrum gefunden werden sollte. Vorteilhafter Weise wird bei dem gegenständlichen Verfahren eine Temperatur von -20˚C bis 100˚C, vorzugsweise 0˚C bis 50˚C, eingestellt. Ganz besonders bevorzugt arbeitet man bei einer Temperatur von $30 \pm 10$˚C.

Das Verhältnis von eingesetztem Substrat zu Katalysator kann vom Fachmann entsprechend den oben angegebenen Randbedingungen gewählt werden. Bevorzugt beträgt das Verhältnis Substrat/Katalysator zwischen 50000: 1 und 10:1, vorzugsweise 1000:1 und 50:1, um ganz besonders bevorzugt zwischen 500:1 und 100:1. Für das erfindungsgemäße Verfahren ist es vorteilhaft, in Gegenwart von Additiven zu Arbeiten. Bevorzugte Additive sind Brönsted Säuren, wie HCl, $HBF_4$ oder Trifluormethansulfonsäure und Lewis Säuren, wie $Ti(O\text{-}i\text{-}Pr)_4$ oder $TiCl_4$, die von 0 - 500 mol-%, bevorzugt 0.1 - 200 mol-%, ganz bevorzugt von 2 - 110 mol-% bezogen auf das eingesetzte Oxim (I) angewandt werden.

Vorteilhaft ist die Ausführungsform, bei der die gegenständliche Erfindung entsprechend der W00384971 mit polymervergrößerten Liganden bzw. Komplexe in einem Membranreaktor durchgeführt wird. Die in dieser Apparatur neben der batch und semikontinuierlichen Fahrweise mögliche kontinuierliche Fahrweise kann dabei wie gewünscht im Cross-Flow-Filtrationsmodus oder als Dead-End-Filtration durchgeführt werden.

Beide Verfahrensvarianten sind prinzipiell im Stand der Technik beschrieben (Engineering Processes for Bioseparations, Ed.: L.R. Weatherley, Heinemann, 1994, 135-165; Wandrey et al., Tetrahedron Asymmetry 1999, 10, 923-928). Bevorzugt führt man die erfindungsgemäße Katalyse kontinuierlich, z.B. in einem Membranreaktor durch.

Damit ein Komplex/Katalysator für den Einsatz in einem Membranreaktor geeignet erscheint, muss er verschiedensten Kriterien genügen. So ist zum einen darauf zu achten, dass ein entsprechend hohes Rückhaltevermögen für den polymervergrößerten Komplex/Katalysator vorhanden sein muss, damit eine zufriedenstellende Aktivität im Reaktor über einen gewünschten Zeitraum gegeben ist, ohne dass fortwährend Komplex/Katalysator nachdosiert werden muss, was betriebswirtschaftlich nachteilig ist (DE19910691). Weiterhin sollte der eingesetzte Katalysator eine angemessene tof (turn over frequency) besitzen, um das Substrat in ökonomisch vernünftigen Zeiträumen in das Produkt umwandeln zu können.

Unter polymervergrößertem Komplex/Katalysator wird im Rahmen der Erfindung die Tatsache verstanden, dass ein oder mehrere aktive die chirale Induktion bedingende Einheiten (Liganden) in dazu geeigneter Form mit weiteren Monomeren copolymerisiert werden oder dass diese Liganden an ein schon vorhandenes Polymer nach dem Fachmann bekannten Methoden angekoppelt werden. Zur Copolymerisation geeignete Formen der Einheiten sind dem Fachmann wohl bekannt und von ihm frei wählbar. Vorzugsweise geht man dabei so vor, dass man das betrachtete Molekül je nach Art der Copolymerisation mit zur Copolymerisation befähigten Gruppen derivatisiert z.B. bei der Copolymerisation mit (Meth)acrylaten durch Ankopplung an Acrylat/Acrylamidmoleküle. In diesem Zusammenhang wird insbesondere auf die EP 1120160 und dort dargestellte Polymervergrößerungen hingewiesen.

Weiterhin, vorteilhaft ist die Ausführungsform, bei der die gegenständliche Erfindung entsprechend (P. Wasserscheid in Ionic Liquids in Synthesis, Wiley-VCH, Weinheim, 2003, pp. 213-257) in Ionischen Flüssigkeiten durchgeführt wird. Als ionische Flüssigkeiten gelten erfindungsgemäß Salze, deren Schmelzpunkt unter 100˚C liegt (P. Wasserscheid, T. Welton, Ionic Liquids in Synthesis, Wiley-VCH, Weinheim, 2003; R. D. Rogers, K. R. Seddon, ACS Symp. Ser., 2002, 818; P. Wasserscheid, W. Keim, Angew. Chem. Int. Ed. 2000, 39, 3772; T. Welton, Chem. Rev. 1999, 99, 2071.)

Die bei der erfindungsgemäßen Reaktion erhaltenen Hydroxylaminderivate können bequem und stereokonservativ in die entsprechenden Amine umgewandelt werden. Dies erfolgt nach dem Fachmann bekannten Methoden (B. Zeeh, H. Metzger in Methoden der Organische Chemie (Houben-Weyl) Bd. X/1, 1971, Georg Thieme Verlag, Stuttgart,

S. 1242-1243). Bevorzugt wird das nach dem erfindungsgemäßen Verfahren hergestellte Hydroxylaminderivat hydrogenolytisch in das Amin gespalten. Die vorstehend beschriebenen erfindungsgemäße Reaktion erlaubt es daher, ausgehend von bestimmten Oximderivaten die in der stereoselektiven Synthese von Wirkstoffen besonders begehrten gegebenenfalls enantiomerenangereicherten Amine als Intermediate herzustellen.

Die bei dem erfindungsgemäßen Verfahren bevorzugt einzusetzenden Übergangsmetallkatalysatoren (Komplexe) sind wie eingangs beschriebenen aus mindestens einem Übergangsmetall und einem oder mehreren Liganden zusammengesetzt.

Geeignete Komplexe sind solche der allgemeinen Formel (V),

$$[M_xP_yL_2S_q]A_r \qquad (V)$$

wobei in der allgemeinen Formel (V) M ein Übergangsmetallzentrum, L gleiche oder verschiedene koordinierende organische oder anorganische Liganden und P bevorzugte Liganden, z.B. Phosphinliganden (s. weiter vorne), darstellen, S koordinierende Lösungsmittelmoleküle und A Äquivalente aus nicht koordinierenden Anionen repräsentiert, wobei x und y ganzen Zahlen größer oder gleich 1, z, q und r ganzen Zahlen größer oder gleich 0 entsprechen.

Die Summe y + z + q wird durch die an den Metallzentren zur Verfügung stehenden Koordinationszentren nach oben begrenzt, wobei nicht alle Koordinationsstellen besetzt sein müssen. Bevorzugt sind Komplexverbindungen mit oktaedrischer, pseudo-oktaedrischer, tetraedrischer, pseudo-tetraedrischer, quadratisch-planarer Koordinationssphäre, die auch verzerrt sein kann, um das jeweilige Übergangsmetallzentrum. Die Summe y + z + q ist in solchen Komplexverbindungen kleiner oder gleich 6.

Die Komplexverbindungen enthalten mindestens ein Übergangsmetallatom oder -ion, insbesondere aus Platin, Palladium, Rhodium, Ruthenium, Osmium, Iridium, Kobalt, Nickel, Eisen oder Kupfer in jedweder katalytisch relevanten Oxidationsstufe.

Bevorzugt sind Komplexverbindungen mit weniger als vier Metallzentren, besonders bevorzugt solche mit ein oder zwei Metallzentren. Die Metallzentren können dabei mit verschiedenen Metallatomen und/oder -ionen besetzt sein.

Bevorzugte Liganden L solcher Komplexverbindungen sind Halogenid, besonders Cl, Br und I, Dien, besonders Cyclooctadien, Cyclopentadienyl, Indenyl und Fluorenyl, Norbornadien, Olefin, besonders Ethylen und Cycloocten, Acetato, Trifluoracetato, Acetylacetonato, Allyl, Methallyl, Alkyl, besonders Methyl und Ethyl, Nitril, besonders Acetonitril und Benzonitril, sowie Carbonyl und Hydrido Liganden.

Bevorzugte koordinierende Lösungsmittel S sind Ether, Amine, besonders Triethylamin, Alkohole, besonders Methanol, Ethanol, i-Propanol und Aromaten, besonders Benzol und Cumol, DMF oder Aceton.

Bevorzugte nichtkoordinierende Anionen A sind Trifluoracetat, Trifluormethansulfonat, $BF_4$, $C10_4$, $PF_6$, $SbF_6$, und $BAr_4$, wobei Ar ($C_6$-$C_{18}$) -Aryl sein kann.

In den einzelnen Komplexverbindungen können dabei unterschiedliche Moleküle, Atome oder Ionen der einzelnen Bestandteile M, P, L, S und A enthalten sein.

Bevorzugt unter den ionisch aufgebauten Komplexverbindungen sind Verbindungen des Typs $[RhP(Dien)]^+A^-$ , wobei P einen erfindungsgemäßen Liganden der Formeln (I) repräsentiert.

Die eben gezeigte exemplarische Herstellung der erfindungsgemäßen Metall-Ligand-Komplexverbindungen kann in situ durch Reaktion eines Metallsalzes oder eines entsprechenden Vorkomplexes mit den Liganden der allgemeinen Formeln (I) erfolgen. Darüber hinaus kann eine Metall-Ligand-Komplexverbindung durch Reaktion eines Metallsalzes oder eines entsprechenden Vorkomplexes mit den Liganden der allgemeinen Formeln (I) und anschließende Isolierung gewonnen werden.

Beispiele für derartige Metallsalze sind Metallchloride, - bromide, -iodide, -cyanide, -nitrate, -acetate, - acetylacetonate, -hexafluoracetylacetonate, tetrafluoroborate, -perfluoracetate oder -triflate, insbesondere des Palladium, Platins, Rhodium, Ruthenium, Osmium, Iridium, Kobalts, Nickels, eisens oder des Kupfers.

Beispiele für die Vorkomplexe sind:

Cyclooctadienplatinchlorid, Cyclooctadienplatiniodid, 1,5-Hexadienplatinchlorid,

1,5-Hexadienplatiniodid, Bis(cyclooctadien)platin, Kalium(ethylentrichloroplatinat),

Cyclooctadienrhodium(I)chlorid-Dimer, Norbornadienrhodium(I)chlorid-Dimer,

1,5-Hexadienrhodium(I)chlorid-Dimer, Tris(triphenylphosphan)rhodium(I)chlorid,

Hydridocarbonyltris(triphenylphosphan)rhodium(I)chlorid,

Bis(norbornadien)rhodium(I)perchlorat, Bis(norbornadien)rhodium(I)tetrafluorborat, Bis(norbornadien)rhodium(I) triflat,

Bis(acetonitrilcyclooctadien)rhodium(I)perchlorat, Bis(acetonitrilcyclooctadien)rhodium(I)tetrafluorborat, Bis(acetonitrilcyclooctadien)rhodium(I)triflat

Bis(acetonitrilcyclooctadien)rhodium(I)perchlorat, Bis(acetonitrilcyclooctadien)rhodium(I)tetrafluorborat, Bis(acetonitrilcyclooctadien)rhodium(I)triflat, 1,5,Cyclooctadien-acetoacetonat-rhodium(I)-salze mit Halogenid, Triflat-, Tetrafluoroborat-, Perchloratanionen, Cyclopentadienrhodium(III)chlorid-Dimer, Pentamethylcyclopentadienrhodium (III)chlorid-Dimer,

(Cyclooctadien)Ru($\eta^3$-allyl)$_2$, ((Cyclooctadien)Ru)$_2$(acetat)$_4$, ((Cyclooctadien)Ru)$_2$(trifluoracetat)$_4$, $RuCl_2$(Aren)-Dimer, $(RuArenI_2)_2$, Tris(triphenylphosphan)ruthenium(II)chlorid, Cyclooctadienruthenium(II)chlorid, $OsCl_2$(Aren)-Dimer, Cyclooctadieniridium(I)chlorid-Dimer, Bis(cycloocten)iridium(I)chlorid-Dimer,

Bis(cyclooctadien)nickel, (Cyclododecatrien)nickel, Tris(norbornen)nickel, Nickeltetracarbonyl, Nickel(II)acetylacetonat,

(Aren)kupfertriflat, (Aren)kupferperchlorat, (Aren)kupfertrifluoracetat, Kobaltcarbonyl.

Die Komplexverbindungen auf Basis von ein oder mehreren Metallen der metallischen Elemente und Liganden der allgemeinen Formel (I), insbesondere aus der Gruppe von Ru, Os, Co, Rh, Ir, Ni, Pt, Pd, Fe, Cu, können bereits Katalysatoren sein oder zur Herstellung von erfindungsgemäßen Katalysatoren auf Basis eines oder mehrerer Metalle der metallischen Elemente, insbesondere aus der Gruppe von Ru, Os, Co, Rh, Ir, Ni, Pd, Fe, Pt, Cu verwendet werden.

Bei der enantioselektiven Hydrierung geht man vorzugsweise so vor, dass man zu hydrierendes Substrat und Übergangsmetallkatalysator in einem Lösungsmittel löst. Vorzugsweise wird der Katalysator wie oben angedeutet aus einem Präkatalysator in Gegenwart des chiralen Liganden durch Reaktion oder durch Vorhydrieren gebildet, bevor das Substrat zugesetzt wird. Anschließend wird bei dem entsprechenden Wasserstoffdruck oder unter Transferbedingungen hydriert.

Die Temperatur bei der Hydrierung sollte so gewählt werden, dass die Reaktion bei den gewünschten Enantiomerenüberschüssen hinreichend schnell verläuft, jedoch Nebenreaktionen möglichst vermieden werden. Zur Einstellung des entsprechenden Substrat/Katalysator-Verhältnis bzw. der einzustellenden Temperatur sei auf oben dargestellte Bereiche verwiesen.

Zum Zeitpunkt der Erfindung hat es mitnichten nahe gelegen, dass das hier vorgestellte Verfahren es gestattet, mit speziellen Katalysatorsystemen und speziellen Oximderivaten eine Hydrierung durchzuführen, welche im Vergleich zum Stand der Technik wesentlich bessere Ausbeuten und bei Einsatz chiraler Liganden bessere Enantiomerenüberschüsse des Reaktionsprodukt bewerkstelligen kann.

Als ($C_1$-$C_8$)-Alkylreste sind anzusehen Methyl, Ethyl, *n*-Propyl, Isopropyl, *n*-Butyl, Isobutyl, sec-Butyl, *tert*-Butyl, Pentyl, Hexyl, Heptyl oder Octyl samt aller ihrer Bindungsisomeren.

Der Rest ($C_1$-$C_8$) -Alkoxy entspricht dem Rest ($C_1$-$C_8$) -Alkyl mit der Maßgabe, dass dieser über ein Sauerstoffatom an das Molekül gebunden ist.

Als ($C_2$-$C_8$)-Alkoxyalkyl sind Reste gemeint, bei denen die Alkylkette durch mindestens eine Sauerstoffunktion unterbrochen ist, wobei nicht zwei Sauerstoffatome miteinander verbunden sein können. Die Anzahl der Kohlenstoffatome gibt die Gesamtzahl der im Rest enthaltenen Kohlenstoffatome an.

Eine ($C_2$-$C_8$)-Alkylenbrücke ist eine Kohlenstoffkette mit drei bis fünf C-Atomen, wobei diese Kette über zwei verschiedene C-Atome an das betrachtete Molekül gebunden ist. Diese kann einen gesättigten oder ungesättigten carbocyclischen oder heterocyclischen Ring bedeuten, mit 1-4 Stickstoff-, Silicium-, Schwefel-, Phosphor- oder Sauerstoffatomen im Ringsystem.

Die in den vorangehenden Absätzen beschriebenen Reste können einfach oder mehrfach mit Halogenen und/oder N-, O-, P-, S-, Si-atomhaltigen Resten substituiert sein. Dies sind insbesondere Alkylreste der oben genannten Art, welche eines oder mehrere dieser Heteroatome in ihrer Kette aufweisen bzw. welche über eines dieser Heteroatome an den Rest gebunden sind.

Unter ($C_3$-$C_8$) -Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-, Si-atomhaltige Reste substituiert sein und/oder N-, O-, P-, S-Atome im Ring aufweisen, wie z. B. 1-, 2-, 3-, 4-Piperidyl, 1-, 2-, 3-Pyrrolidinyl, 2-, 3-Tetrahydrofuryl, 2-, 3-, 4-Morpholinyl.

Ein ($C_3$-$C_8$) -Cycloalkyl- ($C_1$-$C_8$) -Alkylrest bezeichnet einen wie oben dargestellten Cycloalkylrest, welcher über einen wie oben angegebenen Alkylrest an das Molekül gebunden ist.

($C_1$-$C_8$)-Acyloxy bedeutet im Rahmen der Erfindung einen wie oben definierten Alkylrest mit max. 8 C-Atomen, welcher über eine COO-Funktion an das Molekül gebunden ist.

($C_1$-$C_8$)-Acyl bedeutet im Rahmen der Erfindung einen wie oben definierten Alkylrest mit max. 8 C-Atomen, welcher über eine CO-Funktion an das Molekül gebunden ist.

Unter einem ($C_6$-$C_{18}$)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl- , Biphenylreste oder an das betreffende Molekül annelierte Systeme der vorbeschriebenen Art, wie z.B. Indenylsysteme, welche ggf. mit Halogen, ($C_1$-$C_8$) -Alkyl, ($C_1$-$C_8$)-Alkoxy, $NH_2$, NH ($C_1$-$C_8$) -Alkyl, N(($C_1$-$C_8$)-Alkyl)$_2$, OH, $CF_3$, NH($C_1$-$C_8$)-Acyl, N(($C_1$-$C_8$)-Acyl)$_2$, ($C_1$-$C_8$)-Acyl, ($C_1$-$C_8$)-Acyloxy, P(O)$R^{1'}_2$, P(O) ($OR^{1'}$)$_2$, $SO_{(3-1)}$-$R^{1'}$, $SiR^{1'}R^{1'}R^{1'}$ substituiert sein können.

Ein ($C_7$-$C_{19}$) -Aralkylrest ist ein über einen ($C_1$-$C_8$) - Alkylrest an das Molekül gebundener ($C_6$-$C_{18}$)-Arylrest.

Ein ($C_3$-$C_{18}$) -Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Reste angesehen, wie 1-, 2-, 3-Furyl, wie 1-, 2-, 3-Pyrrolyl, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, Acridinyl, Chinolinyl, Phenanthridinyl, 2-, 4-, 5-, 6-Pyrimidinyl. Dieser Rest kann mit den gleichen Resten substituiert sein wie der oben genannte Arylrest.

Unter einem ($C_4$-$C_{19}$)-Heteroaralkyl wird ein dem ($C_7$-$C_{19}$)-Aralkylrest entsprechendes heteroaromatisches System verstanden.

Als Halogene (Hal) kommen Fluor, Chlor, Brom und Iod in Frage.

PEG bedeutet Polyethylenglykol.

Unter dem Begriff enantiomerenangereichert oder Enantiomerenüberschuss wird im Rahmen der Erfindung der Anteil eines Enantiomers im Gemisch mit seiner optischen Antipode in einem Bereich von >50 % und <100 % verstanden. Der ee-Wert berechnet sich wie folgt:

([Enantiomer1]-[Enantiomer2])/([Enantiomer1]+[Enantiomer2])=ee-Wert

Im Rahmen der Erfindung wird unter Membranreaktor jedwedes Reaktionsgefäß verstanden, bei dem der molekulargewichtsvergrößerte Katalysator in einem Reaktor eingeschlossen wird, während niedermolekulare Stoffe dem Reaktor zugeführt werden oder ihn verlassen können. Dabei kann die Membran direkt in den Reaktionsraum integriert werden oder außerhalb in einem separaten Filtrationsmodul eingebaut sein, bei der die Reaktionslösung kontinuierlich oder intermittierend durch das Filtrationsmodul strömt und das Retentat in den Reaktor zurückgeführt wird. Geeignete Ausführungsformen sind u.a. in der WO98/22415 und in Wandrey et al. in Jahrbuch 1998, Verfahrenstechnik und Chemieingenieurwesen, VDI S. 151ff.; Wandrey et al. in Applied Homogeneous Catalysis with Organometallic Compounds, Vol. 2, VCH 1996, S.832 ff.; Kragl et al., Angew. Chem. 1996, 6, 684f. beschrieben.

Unter einem polymervergrößerten Ligand/Komplex ist im Rahmen der Erfindung ein solcher zu verstehen, bei dem das molekulargewichtsvergrößernde Polymer kovalent an den Liganden gebunden ist.

Eine α-Aminosäure ist eine solche ausgewählt aus der Gruppe der Verbindungen, bei denen die Aminofunktion und eine Carbonsäure- oder Carbonsäureesterfunktion über ein C-Atom miteinander verbunden sind. Solche Derivate können natürliche oder nicht in der Natur vorkommende α-Aminosäuren umfassen (EP1024133; Bayer, Walter - Lehrbuch der Organischen Chemie 1991, 11. Auflage, S. 821ff.). β-Aminosäure bezeichnen Verbindungen, bei denen sich 2 C-Atome zwischen der Aminofunktion und der Carbonsäure- oder Carbonsäureesterfunktion befinden.

Experimenteller Teil

Beispiel 1: 3-methoxyamino-buttersäureethylester

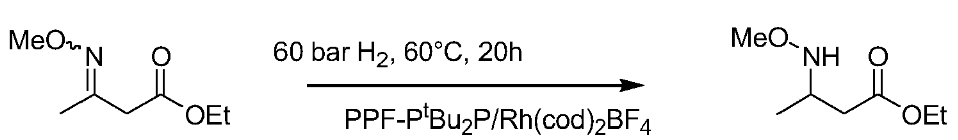

In einem 2 ml Autoklav wurde 3-methoxyiminobuttersäureethylester (80 µl, 0.5 mmol) in 0.8 ml eines Lösungsmittels gelöst. Zugesetzt wurden ggf. (2 mmol) eines Zusatzes und eine Lösung von $[Rh(COD)_2]BF_4$ (6 µmol) und (R)-1-[(S)-2-Diphenylphosphinoferrocenyl]ethyldi-tert.-butylphosphine (10 µmol) in 90 µl Dichlormethan unter Argon. Bei 60˚C und einem Anfangswasserstoffdruck von 60 bar wurde die Mischung gerührt. Nach 20 Stunden wurde entspannt, die Reaktionslösung mit 2 ml Toluol verdünnt und mit wässriger Natriumhydrogencarbonat-Lösung gewaschen. Die organische Phase wurde über $MgSO_4$ getrocknet und über Aluminiumoxyd filtriert. Anschließend wurde die erhaltene Lösung gaschromatographisch untersucht. Die Ergebnisse sind in der Tabelle zusammengefasst:

| Zusatz | Lösungsmittel | Umsatz, % | Produkt, % |
|---|---|---|---|
| | $CH_3COOH$ | 55 | 31 |
| $CF_3COOH$ | $CF_3CH_2OH$ | 100 | 26 |
| $CF_3COOH$ | $CH_3OH$ | 60 | 31 |
| $HBF_4$ | THF | 100 | 65 |

Beispiel 2: 3-methoxyamino-buttersäureethylester

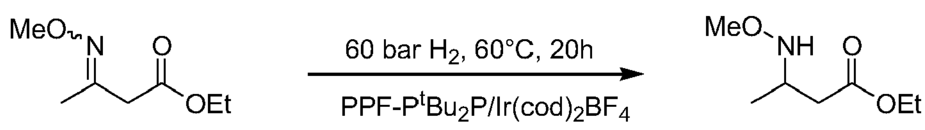


In einem 2 ml Autoklav wurde 3-Methoxyiminobuttersäureethylester (80 µl, 0.5 mmol) in 0.8 ml eines Lösungsmittels gelöst. Der Zusatz (2 mmol) und eine Lösung von $[Ir(COD)_2]BF_4$ (6 µmol) sowie (R)-1-[(S)-2-Diphenylphosphinoferrocenyl]ethyldi-tert.-butylphosphine (10 µmol) wurden in 90 µl Dichlormethan unter Argon gemsicht und bei 60˚C und einem Anfangswasserstoffdruck von 60 bar gerührt. Nach 20 Stunden wurde entspannt, die Reaktionslösung mit 2 ml Toluol verdünnt und mit wässriger Natriumhydrogencarbonat-Lösung gewaschen, die organische Phase über $MgSO_4$ getrocknet und über Aluminiumoxyd filtriert und die Lösungen gaschromatographisch untersucht. Die Ergebnisse sind in der Tabelle zusammengefasst:

| Zusatz | Lösungsmittel | Umsatz, % | Produkt, % |
|---|---|---|---|
| CF3COOH | CF3CH2OH | 95 | 62 |
| CF3COOH | CH30H | 21 | 6 |
| HBF4 | THF | 100 | 79 |

Beispiel 3: 3-methoxyamino-buttersäureethylester

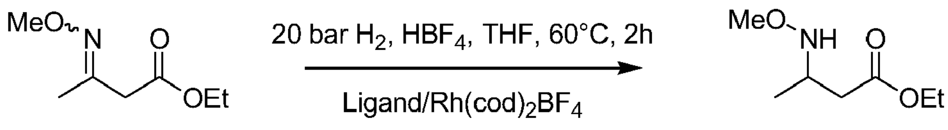


In einem 2 ml Autoklav wurde 3-Methoxyiminobuttersäureethylester (160 µl, 1 mmol) in 0.8 ml eines Lösungsmittels gelöst. Tetrafluoroborsäure 54% in Diethylether (130 µl, 1.04 mmol) und eine Lösung von $[Rh(COD)_2]BF_4$ (6 µmol) und Ligand (10 µmol) wurden in 90 µl Dichlormethan unter Argon hinzugegeben und bei 60˚C und einem Anfangswasserstoffdruck von 20 bar gerührt. Nach 2 Stunden wurde entspannt, die Reaktionslösung mit 2 ml Toluol

verdünnt und mit wässriger Natriumhydrogencarbonat-Lösung gewaschen, die organische Phase über $MgSO_4$ getrocknet und über Aluminiumoxyd filtriert und die Lösungen gaschromatographisch untersucht. Die Enantiomerenüberschüsse wurden mittels HPLC bestimmt: Chiracel OD, Hexan:2-Propanol (99:1), 0.4 ml/min.

Tabelle

| *Ligand* | Umsatz, % | Ee, % |
|---|---|---|
| (R) -1- [(S)-2-Diphenylphosphinoferrocenyl]ethyldi-tert-butylphosphine | 99 | 81 |
| (S)-Diphenylphosphino-2-[ä-(R)-N,N-dimethylamino-(o-diphenylphosphinophenyl)methyl] ferrocen | 99 | 15 |
| (S)-Di(4-methoxyphenyl)phosphino-2-[α-(R)-N,N-dimethylamino-(o-di(4-methoxyphenyl) phophinophenyl)methyl]ferrocen | 61 | 28 |
| (S)-1-(Diphenylphosphino)-2-[α-(R)-(o-diphenylphosphinophenyl)-methoxymethyl]-ferrocene | 98 | 15 |
| (αR, αR)-2,2'-Bis(α-N, N-dimethylaminophenylmethyl)-(S,S)-1,1'-bis(diphenylphosphino) ferrocen | 39 | 1 |
| (1S, 2S, 3R)-1,2-Dimethyl-2,3-bis-(diphenylphosphanylmethyl)-1-hydroxymethylcyclopentan | 68 | 6 |
| (S)-4-(2-Methoxyphenyl)-4,5-dihydro-3H-dinaphtho-[2,1-c;1',2'-e]-phosphepin | 29 | 2 |

Beispiel 4: 3-methoxyamino-buttersäureethylester

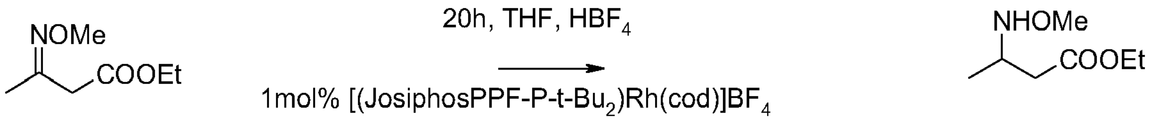


In einem 2 ml Autoklav wurde 3-methoxyiminobuttersäureethylester (160 µl, 1 mmol) in 0.8 ml THF gelöst. Tetrafluoroborsäure 54% in Diethylether (130 µl, 1.04 mmol) und eine Lösung von $[Rh(COD)_2]BF_4$ (6 µmol) und (R)-1-[(S)-2-Diphenylphosphinoferrocenyl]ethyldi-tert.-butylphosphine (10 µmol) in 90 µl Dichlormethan wurden unter Argon hinzugegeben. Der Autoklav wurde nach 20 Stunden entspannt, die Reaktionslösung mit 2 ml Toluol verdünnt und mit wässriger Natriumhydrogencarbonat-Lösung gewaschen, die organische Phase über $MgSO_4$ getrocknet und über Aluminiumoxyd filtriert. Die erhaltene Lösung wurde anschließend gaschromatographisch untersucht. Die Enantiomerenüberschüsse wurden mittels HPLC bestimmt: Chiracel OD, Hexan:2-Propanol (99:1), 0.4 ml/min. Die Ergebnisse sind in der Tabelle zusammengefasst:

| Temp ˚C | H2, bar | Umsatz, % | Ee, % |
|---|---|---|---|
| 25 | 5 | 70 | 92 |
| 25 | 10 | 86 | 91 |
| 25 | 20 | 88 | 93 |

(fortgesetzt)

| Temp ˚C | H2, bar | Umsatz, % | Ee, % |
|---|---|---|---|
| 25 | 40 | 87 | 90 |
| 60 | 5 | 77 | 86 |
| 60 | 10 | 83 | 86 |
| 60 | 20 | 84 | 87 |
| 60 | 40 | 86 | 86 |

Beispiel 5. 3-Phenyl-3-methoxyamino-propionsäureethylester.

In einem 100 ml Parr-Autoklav wurde eine Lösung von 3-Phenyl-3-methoxyimino-propionsäureethylester (6.64 g, 30 mmol) in 40 ml THF gelöst, Tetrafluoroborsäure 54% in Diethylether (5 ml, 40 mmol) und eine Lösung von [Rh (COD)2]BF4 (122 mg, 0.3 mmol) und (R)-1-[(S)-2-Diphenylphosphinoferrocenyl]ethyldi-tert.-butylphosphine (180 mg, 0.33 mmol) in 1 ml Dichlormethan unter Argon hinzugegeben. Bei Raumtemperatur wurde die Mischung bei einem Anfangswasserstoffdruck von 20 bar gerührt. Nach 2 Stunden wurde entspannt, die Reaktionslösung mit 30 ml Toluol verdünnt und mit wässriger Natriumhydrogencarbonat-Lösung gewaschen. Die organische Phase wurde über $MgSO_4$ getrocknet und über Aluminiumoxyd filtriert. Nach Verdampfen des Lösungsmittels wurde der Rückstand (5.7 g) NMR-spectroskopisch und gaschromatographisch untersucht.

NMR (CDC13): 1H $\delta$ = 1.19 (t, *J* = 7.1, 3H), 2.64 (dd, *J* = 15.6, J = 6.0, 1H), 2.87 (dd, J = 15.6, J = 8.0, 1H), 3.44 (s, 3H), 4.092 (q, *J* = 7.1, 1H), 4.094 (q, *J* = 7.1, 1H), 4.45 (dd, *J* = 8.0, *J* = 6.0, 1H), 6.95 (br s, 1H), 7.14-7.31 (m, 5H); 13C $\delta$ = 14.13, 38.92, 60.49, 61.67, 62.34, 125.31, 127.52, 128.23, 140.17, 171.51.

Bei einem Umsatz von 99 % wurde ein Enantiomerenüberschuss von 94% ee festgestellt. Der Ee-Wert wurde mittels HPLC bestimmt: Chiracel OD, Hexan:2-Propanol (95:5), 1 ml/min.

Beispiele 6-8:

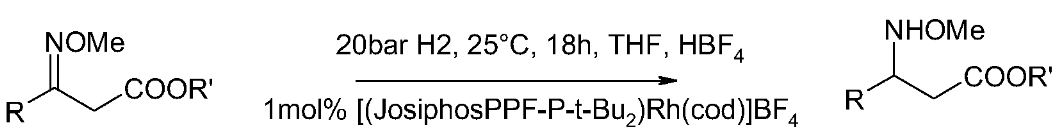


R' = Et, R = Me; R' = Me, R = i-Pr, t-Bu, Ph

Unter gleichen Bedingungen wurden 3-Methoxyiminobuttersäureethylester, 4-Methyl-3-methoxyiminovaleriansäuremethylester und 4,4-Dimethyl-3-methoxyiminovaleriansäuremethylester hydriert. Die Ergebnisse sind in der folgenden Tabelle zusammengefasst.

| R | R' | Umsatz, % | ee, % |
|---|---|---|---|
| Me | Et | 88 | 93 |
| i-Pr | Me | 99 | n.d. |
| t-Bu | Me | 91 | n.d. |

Beispiel 9

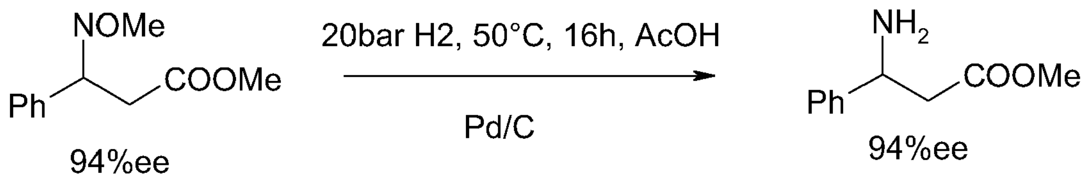


Das Rohprodukt (5.0 g) aus dem Beispiel 5 wurde in 40 ml Eisessig gelöst, mit 1 g 10% Pd/C versetzt und bei 50˚C und einem Anfangswasserstoffdruck von 20 bar gerührt. Nach 16 Stunden wurde die Mischung entspannt, die Reaktionslösung über Celite filtriert und im Vakuum eingeengt. Der Rückstand wurde in 100 ml 2N Salzsäure aufgenommen und mit Diethylether gewaschen. Die wässrige Phase wurde abgetrennt und mit 5% Ammoniak Lösung basisch gestellt. Das Produkt wurde mit Dichlormethan extrahiert und die organische Phase über $MgSO_4$ getrocknet. Nach Verdampfen des Lösungsmittels wurde der Rückstand (1 g) NMR-spectroskopisch und gaschromatographisch untersucht. Bei einer Reinheit von 95% wurde ein Enantiomerenüberschuss von 94% ee festgestellt.

## Patentansprüche

1. Verfahren zur Herstellung von N-Hydroxylaminderivaten durch Hydrierung von Oximderivaten aufweisend ein oder mehrere Strukturelemente der allgemeinen Formel (I)

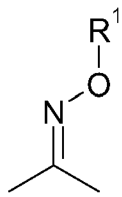

(I)

worin
$R^1$ bzw. $R^{1'}$ bedeutet
($C_1$-$C_8$) -Alkyl, HO- ($C_1$-$C_8$) -Alkyl, ($C_2$-$C_8$) -Alkoxyalkyl, ($C_6$-$C_{18}$) -Aryl, ($C_7$-$C_{19}$) -Aralkyl, ($C_3$-$C_{18}$) -Heteroaryl, ($C_4$-$C_{19}$) -Heteroaralkyl, ($C_1$-$C_8$) -Alkyl- ($C_6$-$C_{18}$) -Aryl, ($C_1$-$C_8$) -Alkyl- ($C_3$-$C_{18}$) -Heteroaryl, ($C_3$-$C_8$) -Cycloalkyl, ($C_1$-$C_8$) -Alkyl- ($C_3$-$C_8$) -Cycloalkyl, ($C_3$-$C_8$) -Cycloalkyl- ($C_1$-$C_8$) -Alkyl, oder
$R^1$ ist C(O)$R^{1'}$ oder C(O)O$R^{1'}$ oder C(O)NH$R^{1'}$ oder C (O) $NR^{1'}_2$ oder P(O)$R^{1'}_2$ oder P(O)(O$R^{1'}$)$_2$ oder $SO_{(3-1)}$-$R^{1'}$ oder Si$R^{1'}R^{1'}R^{1'}$ oder ist ($C_1$-$C_8$)-Alkyl-C(O)$R^{1'}$ oder ($C_1$-$C_8$)-Alkyl-C(O)O$R^{1'}$ oder ($C_1$-$C_8$)-Alkyl-C(O)NH$R^{1'}$ oder ($C_1$-$C_8$)-Alkyl-C(O)N$R^{1'}_2$ oder ($C_1$-$C_8$)-Alkyl-P(O)$R^{1'}_2$ oder ($C_1$-$C_8$)-Alkyl-P(O) (O$R^{1'}$)$_2$ oder ($C_1$-$C_8$)-Alkyl-$SO_{(3-1)}$-$R^{1'}$ oder ($C_1$-$C_8$) -Alkyl-Si$R^{1'}R^{1'}R^{1'}$,
in Gegenwart von homogen löslichen Übergangsmetallkatalysatoren.

2. Verfahren nach Anspruch 1,
**dadurch gekennzeichnet, dass**
man Oxime der allgemeinen Formel (II)

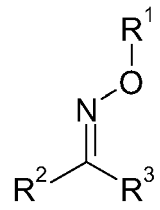

(II)

worin
$R^1$ bevorzugt ($C_1$-$C_8$) -Alkyl bedeutet,
$R^2$ und $R^3$ unabhängig voneinander bedeuten
H, ($C_1$-$C_8$) -Alkyl, HO- ($C_1$-$C_8$) -Alkyl, ($C_2$-$C_8$) -Alkoxyalkyl, ($C_6$-$C_{18}$) -Aryl, ($C_7$-$C_{19}$) -Aralkyl, ($C_3$-$C_{18}$) -Heteroaryl, ($C_4$-$C_{19}$) -Heteroaralkyl, ($C_1$-$C_8$) -Alkyl- ($C_6$-$C_{18}$) -Aryl, ($C_1$-$C_8$) -Alkyl- ($C_3$-$C_{18}$) -Heteroaryl, ($C_3$-$C_8$) -Cycloalkyl, ($C_1$-$C_8$) -Alkyl- ($C_3$-$C_8$) -Cycloalkyl, ($C_3$-$C_8$)-Cycloalkyl-($C_1$-$C_8$) -Alkyl, oder
$R^2$ bzw. $R^3$ bedeuten $C(O)R^{1'}$ oder $C(O)OR^{1'}$ oder $C(O)NHR^{1'}$ oder C (O) $NR^{1'}_2$ oder $P(O)R^{1'}_2$ oder P (O) $(OR^{1'})_{2\ 2}$ oder $SO_{(3-1)}$ -$R^{1'}$ oder $SiR^{1'}R^{1'}R^{1'}$ oder bedeuten ($C_1$-$C_8$) -Alkyl-$C(O)R^{1'}$ oder ($C_1$-$C_8$)-Alkyl-$C(O)OR^{1'}$ oder ($C_1$-$C_8$) -Alkyl-C (O) $NHR^{1'}$ oder ($C_1$-$C_8$)-Alkyl-$C(O)NR^{1'}{}_2$ oder ($C_1$-$C_8$) - Alkyl-$P(O)R^{1'}_2$ oder ($C_1$-$C_8$)-Alkyl-$P(O)(OR^{1'})_2$ oder ($C_1$-$C_8$)-Alkyl-$SO_{(3-1)}$-$R^{1'}$ oder ($C_1$-$C_8$)-Alkyl-$SiR^{1'}R^{1'}R^{1'}$, oder
$R^2$ und $R^3$ und/oder $R^1$ und $R^2$ und/oder $R^1$ und $R^3$ bedeuten eine Verknüpfung durch eine kovalente Bindung,
in die Reaktion einsetzt.

3. Verfahren nach Anspruch 1 und/oder,
**dadurch gekennzeichnet, dass**
man die Hydrierung enantioselektiv durchführt.

4. Verfahren nach Anspruch 1, 2 und/oder 3,
**dadurch gekennzeichnet, dass**
man Übergangsmetalle ausgewählt aus der Gruppe bestehend aus Ru, Os, Co, Rh, Ir, Ni, Pd, Fe, Pt, Cu im Übergangmetallkatalysator einsetzt.

5. Verfahren nach einem oder mehreren der vorhergehenden Ansprüche,
**dadurch gekennzeichnet, dass**
man mittels Hydrierung mit Wasserstoffgas oder mittels Transferhydrierung arbeitet.

6. Verfahren nach Anspruch 5, sofern er sich auf die Hydrierung mit Wasserstoffgas bezieht,
**dadurch gekennzeichnet, dass**
man bei 0,1 bis 100 bar Wasserstoffdruck hydriert.

7. Verfahren nach einem oder mehreren der vorhergehenden Ansprüche,
**dadurch gekennzeichnet, dass**
man bei Temperaturen von -20˚C bis 100˚C, vorzugsweise 0˚C bis 50˚C, arbeitet.

8. Verfahren nach einem oder mehreren der vorhergehenden Ansprüche,
**dadurch gekennzeichnet, dass**
man das Verhältnis Substrat/Katalysator zwischen 50000:1 und 10:1, vorzugsweise 1000:1 und 50:1, wählt.

9. Verfahren nach einem oder mehreren der vorhergehenden Ansprüche,
**dadurch gekennzeichnet, dass**
man in Gegenwart von Additiven, vorzugsweise Brönsted- oder Lewis-Säuren arbeitet.

10. Verfahren nach einem oder mehreren der vorhergehenden Ansprüche,
**dadurch gekennzeichnet, dass**
man die Katalyse kontinuierlich, z.B. in einem Membranreaktor durchführt.

**11.** Verfahren zur Herstellung von enamtiomerenangereichten Aminen,
**dadurch gekennzeichnet, dass**
man das nach einem oder mehreren der vorhergehenden Ansprüche erhaltene N-Hydroxylaminderivat hydrogenolytisch in das Amin spaltet.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0384971 A
- DE 19910691
- EP 1120160 A
- WO 9822415 A
- EP 1024133 A


### In der Beschreibung aufgeführte Nicht-Patentliteratur


- **H. C. J. OTTENHEIM ; J. D. M. HERSCHEID.** *Chem. Rev.,* 1986, vol. 86, 697-707
- **B. ZEEH ; H. METZGER.** Methoden der Organische Chemie. Georg Thieme Verlag, 1971, vol. X / 1, 1092-1279
- **S.-I. MURAHASHI ; S. WATANABE ; T. SHIOTA.** *Chem Commun.,* 1994, 725-726
- **S.-I. MURAHASHI ; T. TSUJI ; S. ITO.** *Chem Commun.,* 2000, 409-410
- **H. METZGER.** Methoden der Organische Chemie. Georg Thieme Verlag, 1968, vol. X / 4, 236-237
- **T. MUNEGUMI ; K. HARADA.** *Bull. Chem. Soc. Jpn.,* 1988, vol. 61, 1425-1427
- **E. BREITNER ; E. ROGINSKI ; P.N. RYLANDER.** *J. Chem. Soc.,* 1959, 2918-2920
- **W. HARTUNG.** *J. Am. Chem. Soc.,* 1928, vol. 50, 3370-3374
- **A.S.C. CHAN ; C.-C. CHEN ; C.-W. LIN ; Y.-C. LIN ; M.-C. CHENG ; S.-M. PENG.** *J. Chem. Soc. Chem. Commun.,* 1995, 1767-8
- **P. KRASIK ; H. ALPER.** *Tetrahedron: Asymmetry,* 1992, vol. 3, 1283-8
- **K. YAMAMOTO.** *Saeed-Ur-Rehman Chem. Lett.,* 1984, 1603-6
- **Y. XIE ; A. MI ; Y. JIANG ; H. LIU.** *Synth. Commun.,* 2001, vol. 31, 2767-2771
- **OJIMA, I.** Catalytic Asymmetric Synthesis. Wiley-VCH Verlag GmbH, 2000
- **BRUNNER, I.** Hydrogenation in Applied Homogeneous Catalysis with organometallic Compounds. Wiley-VCH Verlag GmbH, 2002, 195-212
- **M. WILLS et al.** Asymmetric transferhydrogenation of C=O and C=N bonds. *Tetrahedron: Asymmetry,* 1999, vol. 10, 2045
- **R. NOYORI et al.** Asymmetric transferhydrogenation catalyzed by chiral ruthenium complexes. *Acc. Chem. Res.,* 1997, vol. 30, 97
- **R. NOYORI.** Asymmetric catalysis in organic synthesis. John Wiley & Sons, 1994, 123
- Transition metals for organic Synthesis. Wiley-VCH, 1998, vol. 2, 97
- Comprehensive Asymmetrie Catalysis. Springer-Verlag, 1999
- Comprehensive Asymmetric Catalysis. Springer-Verlag, 1999
- Applied Homogeneous Catalysis with Organometallic Compounds. Willey-VCH, 2002
- Engineering Processes for Bioseparations. Heinemann, 1994, 135-165
- **WANDREY et al.** *Tetrahedron Asymmetry,* 1999, vol. 10, 923-928
- **P. WASSERSCHEID.** Ionic Liquids in Synthesis. Wiley-VCH, 2003, 213-257
- **P. WASSERSCHEID ; T. WELTON.** Ionic Liquids in Synthesis. Wiley-VCH, 2003
- **R. D. ROGERS ; K. R. SEDDON.** *ACS Symp. Ser.,* 2002, 818
- **P. WASSERSCHEID, W. KEIM.** *Angew. Chem. Int. Ed.,* 2000, vol. 39, 3772
- **T. WELTON.** *Chem. Rev.,* 1999, vol. 99, 2071
- **B. ZEEH ; H. METZGER.** Methoden der Organische Chemie. Georg Thieme Verlag, 1971, vol. X/1, 1242-1243
- **WANDREY et al.** Verfahrenstechnik und Chemieingenieurwesen. VDI, 1998, 151ff
- **WANDREY et al.** Applied Homogeneous Catalysis with Organometallic Compounds. VCH, 1996, vol. 2, 832
- **KRAGL et al.** Angew. Chem. 1996, vol. 6, 684f
- **BAYER, WALTER.** Lehrbuch der Organischen Chemie. 1991, 821ff